# FASCICULE DE BREVET EUROPEEN

(11) **EP 1 560 523 B1**
(45) Date de publication et mention de la délivrance du brevet: **24.06.2009**
(21) Numéro de dépôt: 03780291.5
(22) Date de dépôt: 15.10.2003
(51) Int. Cl.: A61B 17/00, A61B 17/12

(54) **DISPOSITIF OCCLUSIF A DESTINATION MEDICALE OU CHIRURGICALE**
VERSCHLUSSVORRICHTUNG FÜR CHIRURGISCHE ODER MEDIZINISCHE ZWECKE
OCCLUSIVE DEVICE FOR MEDICAL OR SURGICAL USE

(30) Priorité: 15.11.2002 FR 0214287
(43) Date de publication de la demande: 10.08.2005
(73) Titulaire: Mialhe, Claude, 83300 Draguignan (FR)
(72) Inventeur: Mialhe, Claude, 83300 Draguignan (FR)
(74) Mandataire: Decobert, Jean-Pascal
(86) Numéro de dépôt international: PCT/FR2003/050092
(87) Numéro de publication internationale: WO 2004/045418

(56) Documents cités:
- EP-A- 0 834 279
- WO-A-02/19926
- WO-A-02/32320
- US-A- 4 580 573

## Description

La présente invention concerne un dispositif occlusif à destination médicale ou chirurgicale, ainsi qu'un dispositif d'occlusion vasculaire et une valve pour instrument chirurgical ou médical.

L'invention trouvera en particulier son application dans le domaine de la fabrication et de la mise en oeuvre de prothèses occlusives pour tous types de vaisseaux dans le corps humain ou animal, prothèses qui comprennent également des dispositifs transpariétaux et endovasculaires.

L'invention concerne également le domaine des instruments chirurgicaux ou médicaux et en particulier des instruments du type introducteurs utilisables lors d'interventions endovasculaires y compris percutanées et/ou transpariétales qui nécessitent la présence d'éléments d'obturation aptes à assurer l'étanchéité de l'introducteur.

La qualité de l'occlusion est un problème constant selon l'état de la technique actuel, que cela soit dans le domaine des prothèses vasculaires ou pour la constitution de valves.

On connaît du document WO-A-0219926, sur lequel est basée la forme en deux parties de la revendication 1, un dispositif d'occlusion vasculaire qui comporte deux organes expansibles pour sa fixation par appui sur deux portions de la paroi du vaisseau, une partie intermédiaire qui est déformable en torsion à un degré ajustable selon la position relative des deux organes expansibles. Une zone de striction maximale est ainsi créée définissant le degré d'occlusion.

Selon ce document, la réalisation d'une obturation totale ou partielle est formée par l'intermédiaire d'une déformation en torsion d'un élément.

Cette technique permet une grande facilité d'intervention et une possibilité de réglage très fin du degré d'obturation.

Il existe cependant un besoin d'améliorer encore l'étanchéité permise par ce type de dispositif.

La présente invention y apporte une solution en adjoignant un autre élément d'occlusion coopérant avec l'élément déformable en torsion.

Dans un mode préféré, l'invention a également l'avantage d'offrir des moyens d'étanchéité additionnels sous forme de joints applicables sur la paroi d'un vaisseau.

Toujours de façon avantageuse, pour la réalisation de dispositif d'occlusion vasculaire, l'invention assure à la fois la constitution d'un élément apte à réaliser l'obturation, ainsi que le guidage par coopération avec un guide amovible présent dans le dispositif au cours de l'intervention.

D'autres buts et avantages apparaîtront au cours de la description qui suit d'un mode préféré de réalisation de l'invention qui n'est cependant pas limitatif.

La présente invention concerne un dispositif occlusif à destination médicale ou chirurgicale comportant un élément cylindrique creux déformable en torsion axiale pour créer une zone de striction. Il comporte un corps déformable en compression transversale, s'appliquant sur la paroi interne de l'élément cylindrique, et comportant un trou débouchant suivant l'axe de l'élément cylindrique.

Suivant des variantes préférées, ce dispositif est tel que :
- Le corps déformable est fixé sur la paroi interne de l'élément cylindrique,
- Le corps déformable est constitué en matériau polymère,
- Il présente deux parties d'extrémité, encadrant l'élément cylindrique et dont la position angulaire relative détermine la torsion dudit élément cylindrique,
- L'élément cylindrique et le corps déformable ont des sections circulaires.

L'invention concerne également un dispositif d'occlusion vasculaire comportant un dispositif occlusif selon l'invention.

Ce dispositif d'occlusion vasculaire est avantageusement réalisé avec les caractéristiques additionnelles suivantes :
- Deux parties d'extrémité, encadrant l'élément cylindrique et dont la position angulaire relative détermine la torsion dudit élément cylindrique, lesdites parties d'extrémité comportant des moyens de fixation sur la paroi d'un vaisseau,
- Les moyens de fixation sont des organes expansibles,
- Il comporte un joint sur la surface extérieure d'au moins un organe expansible, ledit joint étant apte à s'appliquer sur la paroi d'un vaisseau,
- Il présente un voile périphérique d'obturation s'étendant entre une extrémité du corps déformable et la bordure de l'organe expansible correspondant,
- Il comporte un guide amovible orienté suivant l'axe de l'élément cylindrique et traversant le trou du corps déformable,
- Il comporte une gaine amovible intercalée entre la paroi du trou du corps déformable et la paroi externe du guide,
- Il comporte un fourreau amovible entourant le dispositif occlusif,

L'invention concerne également une valve pour instrument chirurgical ou médical comportant un passage obturable et **caractérisée en ce qu**'elle comporte un dispositif occlusif selon l'invention.

Dans un mode préféré de réalisation, cette valve est telle que l'élément cylindrique est déformable en torsion par deux bagues chacune solidaire d'une extrémité de l'élément cylindrique.

Les dessins ci-joints sont donnés à titre d'exemples et ne sont pas limitatifs de l'invention. Ils représentent seulement un mode de réalisation de l'invention et permettront de la comprendre aisément.
La figure 1 est une vue générale du dispositif de l'invention pour une application à l'occlusion vasculaire.
La figure 2 illustre une étape de mise en place d'un dispositif d'occlusion vasculaire de façon transpariétale.
La figure 3 montre un exemple de résultat final d'occlusion transpariétale.
La figure 4 est une vue éclatée de différents éléments constitutifs du dispositif d'occlusion selon l'invention suivant un mode préféré de réalisation.
La figure 5 est une vue de côté d'un corps déformable et la figure 6 en est une vue en coupe.
La figure 7 est une vue de côté du corps déformable en cours de déformation, et la figure 8 en est une vue en coupe.
La figure 9 est une vue de côté du corps déformable en fin de déformation et la figure 10 en est une vue en coupe.
La figure 11 illustre une vue en coupe d'un fourreau utilisable pour un dispositif d'occlusion vasculaire.
La figure 12 présente en coupe longitudinale un dispositif d'occlusion vasculaire.
La figure 13 montre un exemple d'implantation d'un dispositif occlusif selon l'invention dans une valve d'introducteur.

Le dispositif occlusif selon l'invention peut être utilisé dans différents domaines d'applications médicales ou chirurgicales. On décrira plus particulièrement dans la suite de la description un mode de réalisation appliquant le dispositif occlusif à la formation d'un dispositif d'occlusion vasculaire ainsi qu'un mode de réalisation de l'invention appliquant le dispositif occlusif à des valves pour instruments chirurgicaux ou médicaux.

D'une façon générale, le dispositif occlusif selon l'invention comporte un élément cylindrique creux déformable en torsion axiale, cette déformation permettant de créer une zone de striction avantageusement maximale vers le milieu de la longueur de l'élément cylindrique creux bien que ce cas ne soit pas limitatif.

L'élément cylindrique peut être déformé en torsion en modifiant la position angulaire relative de ces extrémités.

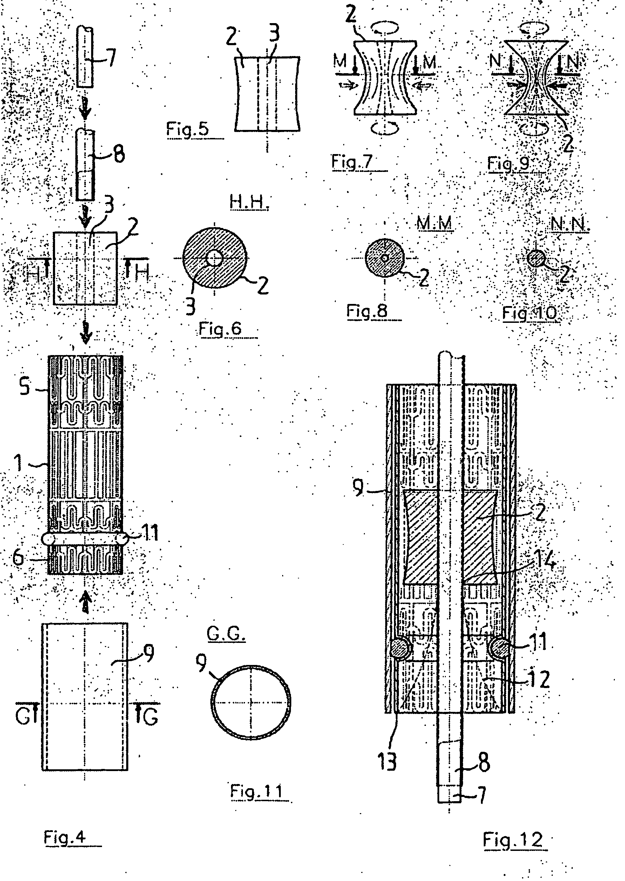

Le dispositif occlusif comporte en outre un corps 2 déformable en compression transversale et apparaissant plus précisément aux figures 4 à 10. Ce corps 2 a des formes et dimensions adaptées pour s'appliquer sur la paroi interne de l'élément cylindrique 1.

A titre préféré, le corps 2 déformable est par ailleurs fixé sur ladite paroi interne de l'élément cylindrique 1 de façon à en suivre les déformations.

En outre, le corps 2 comporte un trou 3 débouchant suivant l'axe 4 de l'élément cylindrique 1.

Le trou 3 offre un passage résiduel apte à être fermé pour réaliser l'obturation. En position ouverte, le trou 3 assure le passage de tout élément fluide ou solide tel qu'un objet allongé dans le cas d'une application à des valves pour instruments chirurgicaux de type introducteur. En ce qui concerne l'application au dispositif d'occlusion vasculaire, le trou 3 permet la réception d'un guide 7 utilisable lors de la manipulation du dispositif d'occlusion au cours de l'intervention chirurgicale.

Différents matériaux peuvent être utilisés pour la constitution du corps 2, et en particulier un matériau polymère, présentant des propriétés de mémoire de forme ou non. D'autres matériaux aptes à se déformer en compression transversale au cours de la torsion de l'élément cylindrique creux 1 pourront être utilisés.

Toujours de façon préférée et en référence aux dessins, le dispositif occlusif de l'invention a une section circulaire sensiblement creuse en particulier en ce qui concerne l'élément cylindrique 1 et le corps déformable 2.

On décrit ci-après plus précisément un mode de réalisation du dispositif occlusif pour une application à un dispositif d'occlusion vasculaire.

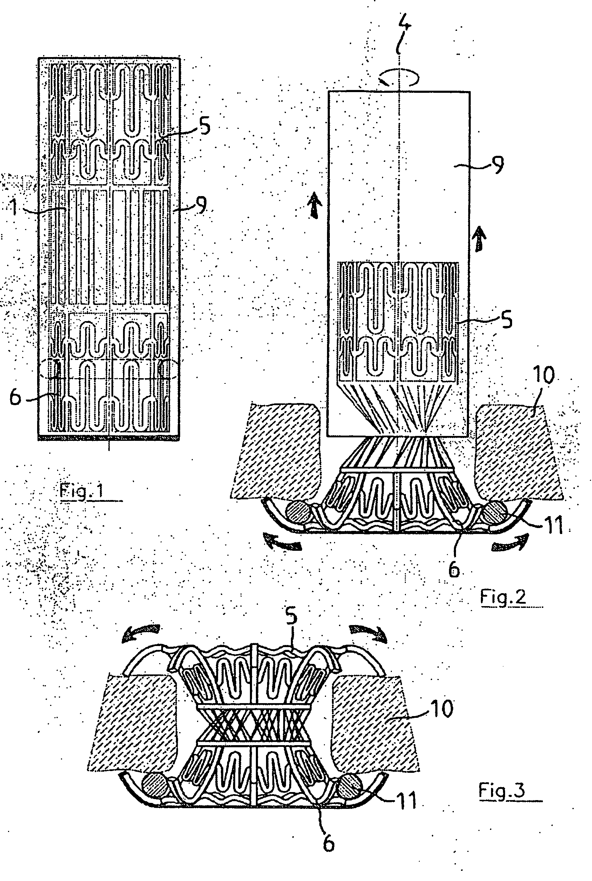

Dans ce cadre, il est fait référence aux figures 1 à 12 présentant un mode particulier de réalisation suivant cette application.

La figure 1 montre en détail un exemple de structure que peut présenter l'élément cylindrique 1. En particulier, l'élément 1 peut se présenter sous la forme d'une armature métallique, par exemple à base de Nitinol ® et présentant trois zones distinctes. La première zone, centrale, constitue l'élément cylindrique 1 en lui-même et est apte à se déformer en torsion tel que cela apparaît en figure 2 et 3. Autour de l'élément cylindrique 1, deux organes expansibles 5 sous forme d'armatures auto expansibles sont représentés et pourront se présenter suivant une configuration telle qu'actuellement utilisée dans le domaine des prothèses endovasculaires. Les organes expansibles 5, 6 ont des propriétés de mémoire de forme aptes à en assurer une déformation par déploiement lorsqu'ils sont libérés.

Cette libération s'effectue par l'intermédiaire d'un fourreau 9 entourant l'ensemble du dispositif avant la mise en place par le praticien. L'élément cylindrique 1 et les organes expansibles 5, 6 sont enveloppés dans le fourreau 9 en position de repos.

Au cours de l'implantation, le praticien retire progressivement le fourreau 9 de façon à libérer d'abord un premier organe expansible pour son application sur la paroi d'un vaisseau 10.

Ce retrait peut être opéré avec l'aide d'un élément pousseur sous forme d'un élément allongé cylindrique creux, apte, par son épaisseur, à s'appliquer sur le bord du dispositif d'occlusion pour exercer un effort contraire au retrait du fourreau 9 et immobiliser le dispositif d'occlusion durant ce retrait.

A cet instant, le dispositif d'occlusion vasculaire est partiellement positionné, mais l'organe expansible 5 reste dans le fourreau 9. La rotation du fourreau 9 par le praticien assure la mise en torsion de l'élément cylindrique 1 et la formation d'une zone de striction, tel que cela est représenté en figure 2.

Lorsque le degré de striction souhaité est obtenu (il est facilement réglable suivant l'amplitude de la rotation opérée par le praticien) l'autre organe expansible 5 est libéré du fourreau 9 par coulissement (toujours éventuellement en combinaison avec l'action d'un pousseur). Cette libération assure son déploiement et son application sur la paroi vasculaire 10.

Les figures 2 et 3 montrent plus particulièrement une application transpariétale au présent dispositif d'occlusion vasculaire. Dans ce cadre, c'est l'organe expansible 6 qui s'applique sur la paroi interne et l'organe expansible 5 sur la paroi externe.

La figure 4 montre plus précisément la coopération des différents éléments constitutifs du dispositif d'occlusion vasculaire.

Dans ce cadre, le fourreau 9 reçoit dans son volume intérieur l'ensemble formé par l'élément cylindrique 1 et les organes expansibles 5, 6. En outre, l'élément cylindrique 1 reçoit dans son volume intérieur pour application sur sa paroi interne un corps déformable 2 présentant un trou débouchant 3 suivant l'axe du dispositif.

Le trou débouchant 3 est quant à lui apte à recevoir un guide 7 utilisable lors des manipulations. A titre préféré, une gaine 8 est positionnée de façon intercalaire entre les parois internes du trou débouchant 3 et la surface périphérique du guide 7. La gaine 8, par exemple en matériau métallique, évite toute détérioration de la matière du corps 2 lors des mouvements du guide 7 et rigidifie l'ensemble constitué. Le guide 7 et la gaine 8 sont constitués de façon amovible de façon à être enlevés par le praticien en cours d'intervention, avant la mise en torsion.

L'association de ces éléments constitutifs est plus particulièrement représentée en figure 12 suivant un mode préféré de réalisation.

Les figures 5 à 10 montrent différents états de sollicitations mécaniques du corps déformable 2. Dans ce cadre, les figures 5 et 6 illustrent respectivement de côté et en coupe la situation du corps 2 en position de repos. Le trou débouchant 3 y est parfaitement libéré.

Au cours de la torsion de l'élément cylindrique 1, le corps 2 est déformé en compression suivant le sens des flèches apparaissant à la figure 7. Le trou débouchant 3 est alors déformé, ce qui réduit son diamètre tel que cela ressort de la figure 8. Cela produit l'obturation, qui peut être totale ou partielle.

En accentuant encore la sollicitation en torsion sur l'élément cylindrique 1, la compression transversale du corps 2 est augmentée jusqu'à parvenir à une obturation complète des trous 3. Cette situation ressort des figures 9 et 10.

A titre préféré, l'étanchéité ainsi obtenue par l'intermédiaire du dispositif occlusif intégré dans le dispositif d'occlusion vasculaire est complétée par des moyens additionnels.

Plus particulièrement, un joint 11 s'appliquant sur la périphérie externe d'un des organes expansibles 6 peut être constitué. On choisira un joint 11 par exemple de forme torique et constitué en une matière suffisamment déformable pour suivre les déformations de l'organe 6 lors de son déploiement.

Le joint 11 s'applique par ce déploiement sur la paroi du vaisseau 10.

Toujours à titre complémentaire à un dispositif occlusif, le dispositif d'occlusion vasculaire peut comprendre un voile 12 tel qu'apparaissant en figure 12. En position de repos, le voile 12 a sensiblement une forme tronconique circulaire et s'étend entre une extrémité 14 du corps 2 et la bordure 13 de l'organe expansible 6 situé du même côté. En constituant un tel voile 12 de façon continue, on forme un effet « entonnoir » évitant toute fuite de flux sanguin en dehors de la zone délimitée par le trou débouchant 3.

Lors du dépliement de l'organe expansible 6 le voile 12 en suit la déformation en corolle.

On décrit ci-après plus précisément un deuxième mode de réalisation du dispositif occlusif de l'invention pour une application à des valves pour instruments chirurgicaux ou médicaux.

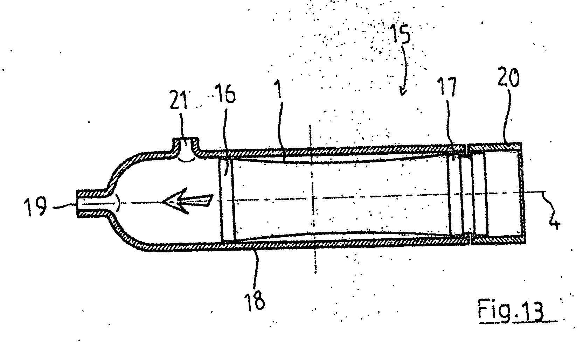

En particulier, la figure 13 illustre la formation d'une telle valve 15 intégrable ou rapportable sur un instrument d'introduction dans le corps.

A cet effet, la valve 15 comprend une partie d'enveloppe 18 apte à recevoir dans son volume intérieur un dispositif occlusif comprenant un élément cylindrique 1.

La valve 15 comprend en outre une extrémité proximale présentant une embouchure 19 pour le passage d'éléments au cours de l'introduction, ainsi qu'une ouverture additionnelle 21.

L'extrémité distale 20 de la valve 15 peut recevoir un élément de valve additionnel ou un simple organe de réglage angulaire.

Suivant cette application, l'élément cylindrique 1 est entouré de bagues 16, 17 dont la position angulaire relative est réglable de façon à assurer la mise en torsion de l'élément 1.

Bien que non représenté, l'élément 1 reçoit dans son volume intérieur un corps déformable 2.

Suivant l'exemple, la rotation de la bague 17 engendrée par manipulation de l'extrémité distale 20 de la valve 15 modifie la position angulaire relative des bagues 16, 17 et assure une déformation en torsion de l'élément 1. Cette déformation en torsion engendre une compression transversale du corps 2 du fait de la striction obtenue.

On peut ainsi ouvrir ou fermer totalement ou non le trou débouchant 3 en jouant sur la position de la bague 17, et ce alors que la bague 16 est fixée.

Bien entendu, ce mode de réalisation n'est qu'indicatif et des variantes peuvent être envisagées.

En particulier, la bague 16 peut être constituée mobile en rotation alors que la bague 17 pourrait être formée de façon fixe. En outre, un mouvement additionnel de rapprochement ou d'éloignement des bagues 16, 17 peut être opéré, par exemple par l'intermédiaire d'une liaison de type glissière entre l'enveloppe 18 de la valve 15 et la bague 17.

Dans le cadre de cette application, l'élément cylindrique 1 comporte une paroi étanche et pourra être constitué notamment en une matière textile tissée ou non, ou encore en matériau polymère tel du P.T.F.E. (Poly Tétra Fluoro Ethylène).

### REFERENCES

- 1.: Elément cylindrique
- 2.: Corps déformable
- 3.: Trou
- 4.: Axe
- 5.: Organe expansible
- 6.: Organe expansible
- 7.: Guide
- 8.: Gaine
- 9.: Fourreau
- 10.: Paroi vasculaire
- 11.: Joint
- 12.: Voile
- 13.: Bordure
- 14.: Extrémité du corps
- 15.: Valve
- 16.: Bague
- 17.: Bague
- 18.: Enveloppe
- 19.: Embouchure
- 20.: Extrémité distale
- 21.: Ouverture

## Revendications

1. Dispositif occlusif à destination médicale ou chirurgicale comportant un élément cylindrique (1) creux déformable en torsion axiale pour créer une zone de striction,
**caractérisé par le fait qu'**il
comporte un corps déformable (2) en compression transversale, s'appliquant sur la paroi interne de l'élément cylindrique (1), et comportant un trou (3) débouchant suivant l'axe (4) de l'élément cylindrique (1).

2. Dispositif selon la revendication 1,
**caractérisé par le fait que**
le corps déformable (2) est fixé sur la paroi interne de l'élément cylindrique (1).

3. Dispositif selon l'une quelconque des revendications 1 à 2,
**caractérisé par le fait que** le
corps déformable (2) est constitué en matériau polymère.

4. Dispositif selon l'une quelconque des revendications 1 à 3,
**caractérisé par le fait qu'**il
présente deux parties d'extrémité, encadrant l'élément cylindrique (1) et dont la position angulaire relative détermine la torsion dudit élément cylindrique (1).

5. Dispositif selon l'une quelconque des revendications 1 à 4
**caractérisé par le fait que**
l'élément cylindrique (1) et le corps déformable (2) ont des sections circulaires.

6. Dispositif d'occlusion vasculaire **caractérisée par le fait qu'**il comporte un dispositif occlusif selon l'une quelconque des revendications 1 à 5.

7. Dispositif selon la revendication 6,
**caractérisé par le fait qu'**il présente
deux parties d'extrémité, encadrant l'élément cylindrique (1) et dont la position angulaire relative détermine la torsion dudit élément cylindrique (1), lesdites parties d'extrémité comportant des moyens de fixation sur la paroi d'un vaisseau.

8. Dispositif selon la revendication 7,
**caractérisé par le fait que**
les moyens de fixation sont des organes expansibles (5, 6).

9. Dispositif selon la revendication 8,
**caractérisé par le fait qu'**il
comporte un joint (11) sur la surface extérieure d'au moins un organe expansible (5, 6), ledit joint (11) étant apte à s'appliquer sur la paroi d'un vaisseau.

10. Dispositif selon l'une quelconque des revendications 8 ou 9,
**caractérisé par le fait qu'**il
présente un voile (12) périphérique d'obturation s'étendant entre une extrémité du corps (14) déformable (2) et la bordure (13) de l'organe expansible (5, 6) correspondant.

11. Dispositif selon l'une quelconque des revendications 6 à 10,
**caractérisé par le fait qu'**il
comporte un guide (7) amovible orienté suivant l'axe (4) de l'élément cylindrique (1) et traversant le trou (3) du corps déformable (2).

12. Dispositif selon la revendication 11,
**caractérisé par le fait qu'**il
comporte une gaine (8) amovible intercalée entre la paroi du trou (3) du corps déformable (2) et la paroi externe du guide (7).

13. Dispositif selon l'une quelconque des revendications 6 à 12,
**caractérisé par le fait qu'**il
comporte un fourreau (9) amovible entourant le dispositif occlusif.

14. Valve (15) pour instrument chirurgical ou médical, comportant un passage obturable,
**caractérisée par le fait que**
elle comporte un dispositif occlusif selon l'une quelconque des revendications 1 à 5.

15. Valve (15) selon la revendication 14,
**caractérisée par le fait que**
l'élément cylindrique (1) est déformable en torsion par deux bagues (16,17) chacune solidaire d'une extrémité de l'élément cylindrique (1).

## Claims

1. Occlusive device for medical or surgical use, comprising a hollow cylindrical element (1) that can be twisted according to its axis to create a striction zone,
**characterised in that** it comprises a transverse compression deformable body (2) applied to the inner wall of the cylindrical element (1), and comprising a through hole (3) according to axis (4) of the cylindrical element (1).

2. Device according to claim 1,
**characterised in that** the deformable body (2) is attached to the inner wall of the cylindrical element (1),

3. Device according to any of claims 1 to 2,
**characterised in that** the deformable body (2) is made from a polymer material,

4. Device according to any of claims 1 to 3,
**characterised in that** there are two end parts, surrounding the cylindrical element (1) and whose angular position determines the torsion of said cylindrical element (1).

5. Device according to any of claims 1 to 4,
**characterised in that** the cylindrical element (1) and the deformable body (2) both have circular cross sections.

6. Vascular occlusion device,
**characterised in that** it comprises an occlusive device according to any of claims 1 to 5.

7. Device according to claim 6,
**characterised in that** it possesses, two end parts, surrounding the cylindrical element (1) and whose relative angular position determines the torsion of said cylindrical element (1), said end parts possessing means of attachment to the wall of a vessel.

8. Device according to claim 7,
**characterised in that** the attachment means are expanding elements (5 and 6).

9. Device according to claim 8,
**characterised in that** it comprises a seal (11) on the outer surface of at least one of the expanding elements (5 and 6), said seal (11) being appropriate for application to the wall of a vessel.

10. Device according to either of claims 8 or 9,
**characterised in that** it presents a peripheral obturation web (12) extending from one end of the deformable (2) body (14) and the edge (13) of the expanding element (5, 6).

11. Device according to any of claims 6 to 10,
**characterised in that** it comprises a removable guide (7) positioned according to the axis (4) of the cylindrical element (1) and crossing the hole (3) in the deformable body (2).

12. Device according to claim 11,
**characterised in that** it possesses a removable sheath (8) inserted between the wall of the hole (3) in the deformable body (2) and the outer wall of the guide (7),

13. Device according to any of claims 6 to 12,
**characterised in that** it comprises a removable sleeve (9) surrounding the occlusive device.

14. Valve (15) for surgical or medical instrument, comprising a closeable passage,
**characterised in that** it comprises an occlusive device according to any of claims 1 to 5.

15. Valve (15) according to claim 14,
**characterised in that** the cylindrical element (1) can be twisted by means of two rings (16 and 17), each of which is integral to one end of the cylindrical element (1).

## Patentansprüche

1. Verschlussvorrichtung für medizinische oder chirurgische Zwecke, bestehend aus einem hohlen, zylindrischen Element (1), das durch Torsion um die Längsachse eine Querschnittsverengung bilden kann, **gekennzeichnet dadurch, dass** es einen unter Druck in Querrichtung verformbaren Körper (2) besitzt, der sich an die Innenwand des zylindrischen Körpers (1) anlegt und eine Öffnung (3) besitzt, die in Richtung der Achse (4) des zylindrischen Elements (1) mündet.

2. Vorrichtung gemäß Patentanspruch 1, **gekennzeichnet dadurch, dass** der verformbare Körper (2) an der Innenwand des zylindrischen Elements (1) fixiert ist.

3. Vorrichtung gemäß einem der Ansprüche 1 bis 2, **gekennzeichnet dadurch, dass** der verformbare Körper (2) aus Polymerwerkstoff besteht.

4. Vorrichtung gemäß einem der Ansprüche 1 bis 3, **gekennzeichnet dadurch, dass** sie zwei Endstücke besitzt, die das zylindrische Element (1) einschließen und deren relative Winkelstellung die Torsion des zylindrischen Elements (1) bestimmt.

5. Vorrichtung gemäß einem der Ansprüche 1 bis 4, **gekennzeichnet dadurch, dass** das zylindrische Element (1) und der verformbare Körper (2) ringförmige Abschnitte besitzen.

6. Verschlussvorrichtung für Blutgefäße, **gekennzeichnet dadurch, dass** sie eine Verschlussvorrichtung gemäß einem der Ansprüche 1 bis 5 besitzt.

7. Vorrichtung gemäß Anspruch 6, **gekennzeichnet dadurch, dass** sie zwei Endstücke besitzt, die das zylindrische Element (1) einschließen und deren relative Winkelstellung die Torsion des zylindrischen Elements (1) bestimmt, wobei die Endstücke Mittel zur Fixierung an der Wand eines Blutgefäßes besitzen.

8. Vorrichtung gemäß Anspruch 7, **gekennzeichnet dadurch, dass** die Fixiermittel expandierbare Organe (5, 6) sind.

9. Vorrichtung gemäß Anspruch 8, **gekennzeichnet dadurch, dass** sie eine Dichtung (11) an der Außenseite mindestens eines der expandierbaren Organe (5, 6) besitzt und diese Dichtung (11) sich an die Wand eines Blutgefäßes anlegen kann.

10. Vorrichtung gemäß einem der Ansprüche 8 oder 9, **gekennzeichnet dadurch, dass** sie an ihrem Umfang eine verschließende Einlage (12) besitzt, die sich von einem Ende des verformbaren (2) Körpers (14) bis zum Rand (13) des entsprechenden expandierbaren Organs (5, 6) erstreckt.

11. Vorrichtung gemäß einem der Ansprüche 6 bis 10, **gekennzeichnet dadurch, dass** sie eine abnehmbare, in Längsrichtung (4) des zylindrischen Elements (1) gerichtete und durch die Öffnung (3) des verformbaren Körpers (2) hindurchtretende Führung (7) besitzt.

12. Vorrichtung gemäß Patentanspruch 11, **gekennzeichnet dadurch, dass** sie ein abnehmbares Schutzrohr (8) zwischen der Wand der Öffnung (3) des verformbaren Körpers (2) und der Außenwand der Führung (7) besitzt.

13. Vorrichtung gemäß einem der Ansprüche 6 bis 12, **gekennzeichnet dadurch, dass** sie eine die Verschlussvorrichtung umgebende, abnehmbare Hülse (9) besitzt.

14. Kappe (15) für chirurgische oder medizinische Instrumente mit einem verschließbaren Durchgang, **gekennzeichnet dadurch, dass** er eine Verschlussvorrichtung gemäß einem der Ansprüche 1 bis 5 besitzt.

15. Klappe (15) gemäß Anspruch 14, **gekennzeichnet dadurch, dass** das zylindrische Element (1) mit Hilfe zweier Ringe (16, 17), der jeder mit einem Ende des zylindrischen Elements (1) verbunden ist, durch Torsion verformbar ist.
